# EUROPÄISCHE PATENTSCHRIFT

(11) **EP 1 915 104 B1**
(45) Veröffentlichungstag und Bekanntmachung des Hinweises auf die Patenterteilung: **09.05.2018**
(21) Anmeldenummer: 06776928.1
(22) Anmeldetag: 17.08.2006
(51) Int. Cl.: A61C 19/045

(54) **Verfahren zum Ermitteln der Zentrikposition eines menschlichen Gebisses**
Method for determining the centric position of human denture
Procédé permettant de déterminer la position centrale d'une denture humaine

(30) Priorität: 17.08.2005 DE 102005038898
(43) Veröffentlichungstag der Anmeldung: 30.04.2008
(73) Patentinhaber: Kaltenbach & Voigt GmbH, 88400 Biberach (DE)
(72) Erfinder: LANG, Hans, Walter, 88299 Leutkirch (DE)
(74) Vertreter: Thun, Clemens
(86) Internationale Anmeldenummer: PCT/EP2006/008129
(87) Internationale Veröffentlichungsnummer: WO 2007/020091

(56) Entgegenhaltungen:
- WO-A-99/15100
- BE-A6- 1 003 824
- DE-A1- 19 956 876
- DE-U1-202004 004 955

## Beschreibung

Die Erfindung findet Anwendung im Dentalbereich, insbesondere bei der Herstellung von Zahnersatz.

Die am weitesten verbreitete Technik zur Herstellung von Zahnersatz ist folgende. Der Zahnarzt nimmt von den Zähnen des Oberkiefers und des Unterkiefers des Patienten Abdrücke. Aus den Abdrücken werden Gipsmodelle des Oberkiefers und des Unterkiefers hergestellt. Diese werden in einen Artikulator eingesetzt, der aus zwei Teilen besteht, die durch zwei Gelenke miteinander verbunden sind und das menschliche Gebiss simuliert. Die beiden Gelenke entsprechen den menschlichen Kiefergelenken. Nach dem Einsetzen der Gipsmodelle müssen die Gelenke patientenspezifisch justiert werden.

Ein bekanntes Verfahren zum Ermitteln der Zentrikposition besteht darin, dass zwischen die geöffneten Zähe des Patienten ein plastisch verformbares plattenförmiges Registrat eingelegt wird und dass der Patient dann zum Zubeißen veranlasst wird. In dem Registrat werden dadurch Abdrücke der Zähne des Oberkiefers und des Unterkiefers eingeformt. Das Registrat wird nach dem Aushärten zwischen die in einen Registrator eingesetzten Gipsmodelle des Ober- und Unterkiefers eingesetzt. Der Unterkiefer wird dann durch Justieren der beiden die Kiefergelenke repräsentierenden Gelenke des Artikulators gegenüber dem Oberkiefer solange verschoben, bis die Zähne der Gipsmodelle des Ober- und Unterkiefers in den Abdrücken auf beiden Seiten des Registrats mit Passsitz eingreifen.

Eine andere ebenfalls bekannte Methode besteht darin, dass in der Mundhöhle des Patienten an den Zähnen des Oberkiefers und des Unterkiefers mittels einer plastisch verformbaren Masse jeweils eine Platte befestigt wird. Die beiden Platten erstrecken sich quer durch die Mundhöhle. Die auf den Zähnen des Unterkiefers befestigte Platte trägt in der Mitte einen nach oben ragenden Stützstift. Die an den Zähnen des Oberkiefers befestigte Platte weist an ihrer Unterseite eine Wachsschicht auf, in der der Stützstift beim Auftreffen einen Abdruck erzeugt. Der Abdruck definiert die Zentrikposition. Die beiden Platten werden dann aus dem Mund des Patienten entfernt und auf die Zähne der Gipsmodelle im Artikulator aufgesetzt. Die Gelenke des Artikulators werden dann so verschoben, dass der Stützstift wieder in den Abdruck der Wachsschicht an der Unterseite der oberen Platte eintaucht. Der Artikulator ist damit auf die Zentrikposition einjustiert. Die Zentrikposition ist hier also eine Referenzposition, die im Patientenmund fixiert und im Artikulator zur Justage verwendet wird.

Eine dritte bekannte Methode zur Ermittlung der Zentrikposition ist eine Abwandlung der zuletzt beschriebenen zweiten Methode. Hier wird der Patient nicht nur zum einmaligen Zubeißen veranlasst, sondern sein Unterkiefer soll eine Vor- und Zurückbewegung sowie Seitenbewegungen nach zwei Seiten ausführen. Dadurch entsteht eine T-artige Spur in der Wachsschicht der oberen Platte, wobei der obere T-Schenkel nach oben durchgewölbt ist. Diese Wölbung nennt man gotischen Bogen. Der Schnittpunkt der beiden T-Schenkel wird nunmehr als derjenige ausgewählt auf den der Stützstift im Artikulator zum Einstellen der Zentrikposition ausgerichtet wird. Bekannt ist ferner ein von der Anmelderin unter der Handelsbezeichnung "Digma bzw. ARCUS-Digma" vertriebenes Gerät. Dieses Gerät ist ein Hilfsmittel zur Einstellung von Artikulatoren. Es führt dazu eine elektronische Positionsanalyse des Unterkiefers durch. Um die Bewegungen des Unterkiefers gegenüber dem Oberkiefer zu messen, wird mit dem Oberkiefer und dem Unterkiefer jeweils ein Trägergestell verbunden. Das mit dem Oberkiefer am oberen Teil des Kopfes verbundenen Trägergestell hat vier Mikrofone; das mit dem Unterkiefer verbundene Trägergestell hat drei Ultraschallsender. Die zwölf Strecken zwischen den drei Sendern und den vier Mikrofonen werden bei Bewegung des Unterkiefers hinsichtlich ihrer Längenänderung ausgewertet. Das Gerät hat bisher keine Anwendung zur Ermittlung der Zentrikposition gefunden. DE 20 2004 004955 U1 offenbart ein Verfahren zum Ermitteln der Zentrikposition eines menschlichen Gebisses nach dem Oberbegriff von Ansprüchen 1 und 3. Die Erfindung hat sich die Aufgabe gestellt, das nachfolgend geschilderte Problem zu lösen. Wenn ein Patient den Unterkiefer gegen den Oberkiefer bewegt und die Zähne aufeinandertreffen, so bedeutet dies nicht zwangsläufig, dass der Unterkiefer in Bezug auf der Oberkiefer stets die gleiche Position einnimmt. Die genannte Position differiert vielmehr in Abhängigkeit von der Spannung der Kaumuskeln, von Signalen, die die Zähne beim Aufeinandertreffen abgegeben, vom Druck auf die Minisken in den Kiefergelenken und von unterschiedlichen Signalen aus dem Gehirn. Letztere werden dadurch beeinflusst, dass der Patient ermüdet ist oder unter Spannung steht. Die bisherigen Methoden zur Festlegung der Zentrikposition ist demnach ungenau und zwar deshalb, weil der einmalige Biss oder einmalige Bewegungen von Zufälligkeiten geprägt ist/sind. Der Erfindung liegt die Aufgabe zugrunde, die Genauigkeit des Verfahrens zur Ermittlung der Zentrikposition zu verbessern.

Ausgehend von der zweiten bekannten Methode zum Ermitteln der Zentrikposition besteht eine erste erfindungsgemäße Lösung gemäß Anspruch 1 darin, dass mehrere Beißbewegungen durchgeführt werden, dass die Beißbewegungen messtechnisch erfasst und in elektrische Signaldaten umgewandelt werden, und dass aus den Signaldaten mehrere Schlussstellungen die Zentrikposition ermittelt wird.

Die Zentrikposition wird erfindungsgemäß durch Mittelwertbildung der Signaldaten der mehreren Schlussstellungen in einer Koordinatenebene ermittelt.

Eine andere Möglichkeit besteht darin, dass zur Ermittlung der Zentrikposition nur die in einer Koordinaten-Ebene in einem begrenzten Trefferfeld liegenden Signaldaten ausgewertet werden.

Ausgehend von der oben beschriebenen dritten bekannten Methode für ein Verfahren zum Ermitteln der Zentrikposition eines menschlichen Gebisses, bei dem der Unterkiefer zu mindestens einer Bewegung in Bezug auf den Oberkiefer veranlasst wird, und bei dem mindestens zwei Bewegungen zur Ermittlung der Zentrikposition ausgewertet werden, besteht ein weiterer erfindungsgemäßer Vorschlag zur Lösung der Aufgabe darin, dass der Unterkiefer in der Beißendstellung zu mehreren zwischen der vorderen und der hinteren Endposition verlaufenden ersten Verschiebebewegungen sowie zu mehreren zwischen den beiden seitlichen Endpositionen verlaufenden zweiten Verschiebebewegungen veranlasst wird, dass die ersten und zweiten Verschiebebewegungen messtechnisch erfasst und in elektrische Signaldaten umgewandelt werden, und dass die Signaldaten dahingehend ausgewertet werden, dass der Schnittpunkt der ersten und der zweiten Verschiebebewegungen als Zentrikposition festgesetzt wird. Die Signaldaten der ersten und der zweiten Verschiebebewegung sowie die Signaldaten der ermittelten Zentrikposition können auf einem Display als Symbole, wie z.B. Punkte dargestellt werden. Die Darstellung kann in einer Koordinatenebene erfolgen, wobei die Koordinaten kartesische Koordinaten sind.

Eine bestimmte Zentrikposition muss nicht zwingend mit bestimmten Positionen der beiden Kieferngelenke gekoppelt sein. Durch unsymmetrisches Zubeißen des Patienten kann eine bestimmte Zentrikposition auch unterschiedlichen Paaren von Kieferngelenkpositionen entsprechen. Will man diese Unschärfe ausschalten, müssen für die Justage des Artikulators weitere Informationen zur Verfügung stehen. Das ist beispielsweise der Fall, wenn die Zentrikposition zum Programmieren (Einfügen von Zahnabdrücken) eines Registrats verwendet wird. Dazu bewegt der Patient ggf. mit führender Unterstützung des Zahnarztes seinen Unterkiefer in die ermittelte Zentrikposition, so dass die Zähne von Ober- und Unterkiefer in einem zwischen diese eingelegtes plastisches Registrat Abdrücke produzieren. Die Abdrücke enthalten die zusätzlichen Informationen über die Kiefergelenkpositionen. Das Registrat kann dann entsprechend der zuerst beschriebenen bekannten Methode im Artikulator verwendet werden, um dessen Gelenke zu justieren.

Alternativ dazu ist es möglich, neben den oder anstelle der Signaldaten für die eine Zentrikposition die entsprechenden Signaldaten für eine mittlere Position der beiden Kieferngelenke zu ermitteln. Wenn diese zur Verfügung stehen, kann auf die Verwendung eines Registrats verzichtet werden. Die mittlere Position der beiden Kieferngelenke kann dann direkt zur Justage der beiden Gelenke des Artikulators verwendet werden, entweder von Hand oder motorisch.

Eine Weiterbildung des ersten Lösungsvorschlages kann darin bestehen, dass die Signaldaten der Beißendstellungen aller Beißbewegungen und die Signaldaten der ermittelten Zentrikposition auf einem Display als Symbole, wie z.B. Punkte, dargestellt werden. Die Darstellung kann in einer Koordinatenebene erfolgen, welche die tatsächlichen Abstände der Beißendstellungen von der Zentrikposition in entsprechenden Maßeinheiten erkennen lässt. Vorzugsweise werden hierfür kartesische Koordinaten verwendet.

Eine zweckmäßige Weiterbildung des zweiten erfindungsgemäßen Lösungsvorschlags kann darin bestehen, dass die Signaldaten der der Zentrikposition entsprechenden Kiefergelenkpositionen auf einem Display als Symbole, wie z.B. Punkte, in je zwei Koordinatenebenen dargestellt werden, und dass die Koordinatenebenen einen Winkel, vorzugsweise einen solchen von 90° einschließen. Die eine Koordinatenebene kann bspw. diejenige Schnittebene durch den Patientenkopf repräsentieren, die einerseits zwischen oben (cranial) und unten (cardial) und andererseits vorn (ventral) und hinten (dorsal) verläuft, und dass die andere Koordinatenebene diejenige Schnittebene durch den Patientenkopf repräsentiert, die einerseits zwischen vorn (ventral) und hinten (dorsal) und andererseits zwischen der Körperachse (medial) und der entsprechenden Seite (lateral) verläuft. Die Koordinaten können nach Art von Polarkoordinaten in Form von konzentrischen Ringen dargestellt werden. Dabei können die Symbole für die Kiefergelenkpositionen, welche der Zentrikposition entsprechen, bei Schmerzfreiheit der Kieferngelenke als Referenz für alle anderen möglichen Kiefergelenkpositionen in das Zentrum der Polarkoordinaten verschoben werden.

Die Vorrichtung zur Durchführung eines der zuvor beschriebenen Verfahren zum Ermitteln der Zentrikposition eines menschlichen Gebisses kann wie das bekannte Digma-Gerät Trägergestelle für Geber- und Empfängerelemente aufweisen, wobei je ein Trägergestell in einer reproduzierbaren Position mit dem Unterkiefer und dem Oberkiefer des betreffenden Patienten verbindbar ist und wobei die Entfernungen zwischen den Sender- und Empfängerelementen gemessen und zur Gewinnung von Signaldaten ausgewertet werden.

Es hat sich dabei herausgestellt, dass die Genauigkeit der bei dem bekannten Digma-Gerät vorgesehen vier Geberelemente und drei Empfängerelemente nicht ausreicht, um die gewünschte Genauigkeit zur Bestimmung der Zentrikposition zu gewährleisten. Die erfindungsgemäße Vorrichtung unterscheidet sich daher von dem bekannten Digma-Gerät u.a. darin, dass mehr als drei Geberelemente und mehr als vier Empfängerelemente vorgesehen sind. Weitere gegenständliche Unterschiede ergeben sich aus den entsprechenden Mitteln, mit denen die einzelnen Verfahrensschritte zu realisieren sind. Diese Mittel ergeben sich aus den Verfahrensschritten (z.B. Mittel zur Berechnung des Mittelwertes aus den Signaldaten usw.).

Die Erfindung wird nachfolgend anhand der Zeichnungen erläutert.
- Fig. 1: zeigt einen Artikulator mit eingesetzten Gipsmodellen und Registrat;
- Fig. 2: zeigt den Kopf eines Patienten mit angesetzten Trägerelementen für die Geber- und Sensorelemente;
- Fig. 3: zeigt ein Gerät zur Zentrikbestimmung;
- Fig. 4: zeigt ein Display mit verschiedenen Treffern zur Auswahl der Zentrikposition;
- Fig. 5: zeigt ein Display wie in Fig. 4 zusätzlich mit vier Polarkoordinatenebenen zur Anzeige der getesteten Kiefergelenkpositionen;
- Fig. 6: zeigt ein Display wie in Fig. 4, bei dem jedoch eine andere Methode zur Zentrikbestimmung gewählt wurde.

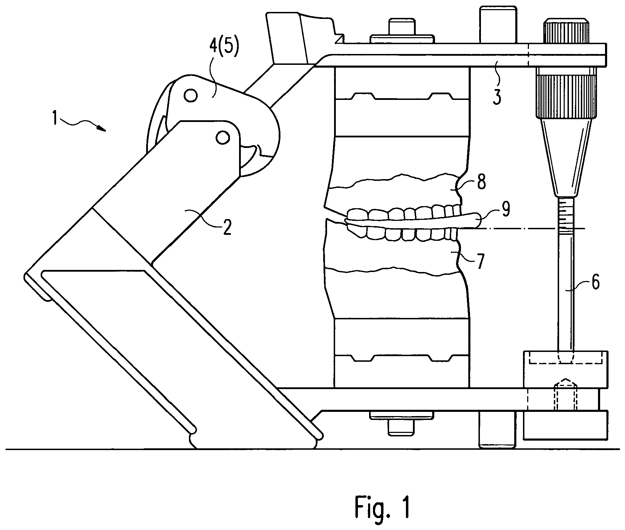

Der in Fig. 1 gezeigte Artikulator 1 ist handelsüblich und z.B. in der DE 42 11 020 C2 beschrieben. Er besteht aus zwei Bügeln 2, 3, die durch Gelenke 4, 5 miteinander verbunden sind, von denen hier nur eines sichtbar ist. Die Gelenke 4, 5 sind zur Einstellung der Zentrikposition verstellbar. Der Abstand der Bügel ist mit einem Stützstift 6 regulierbar. Auf dem Bügel 2 ist das Gipsmodell 7 des Unterkiefers eines Patienten befestigt. Auf dem Bügel 3 ist das Gipsmodell 8 des Oberkiefers des Patienten befestigt. Zwischen den Zähnen der beiden Gipsmodelle 7, 8 befindet sich ein Registrat 9 aus einem aushärtbaren, zunächst plastisch verformbaren Material.

Zur Anfertigung der beiden Gipsmodelle 7, 8 muss der Zahnarzt zunächst Abdrücke von den Zähnen des Oberkiefers und des Unterkiefers nehmen. Er verwendet dazu an die Zahnreihenform angepasste Schalen, in denen sich eine plastisch verformbare Masse befindet, die der Zahnarzt auf die Zähne presst. In die dadurch entstandene Gussform wird dann flüssiger Gips eingegossen, der nach dem Aushärten Gipsmodelle vom Oberkiefer und vom Unterkiefer des Patienten ergibt. Der Patient wird danach bei der herkömmlichen Methode zur Ermittlung der Zentrikposition vom Zahnarzt veranlasst, den Unterkiefer in Form einer einmaligen Beißbewegung gegen den Oberkiefer zu bewegen, so dass in dem Registrat 9 Abdrücke der Zähne des Oberkiefers und des Unterkiefers eingeformt werden. Das Substrat 9 wird dann nach dem Aushärten in den Artikulator - wie in Fig. 1 dargestellt - zwischen die Zähne der beiden Gipsmodelle 7, 8 eingesetzt. Um zu gewährleisten, dass der Artikulator die Parameter des Gebisses des Patienten möglichst weitgehend simuliert, werden nunmehr die Gelenke 4, 5 des Artikulators und der Distanzstift 6 so justiert, dass die Zähne der beiden Gipsmodelle 7, 8 formschlüssig in die Abdrücke des Registrats 9 eingreifen. Wenn diese Justierung vollzogen ist, können die Gipsmodelle in herkömmlicher Weise zur Herstellung von Zahnersatz bearbeitet werden.

Es hat sich aber herausgestellt, dass die bisherige Methode der Festlegung der Zentrikposition ungenau ist, weil der einmalige Biss von Zufälligkeiten geprägt ist, bspw. vom Spannungszustand des Patienten, von der Empfindlichkeit seiner Zähne beim Aufeinandertreffen mit den Gegenzähnen sowie vom Zustand der Minisken in den Kiefergelenken.

Zur Erhöhung der Genauigkeit bei der Ermittlung der Zentrikposition wird deshalb einerseits ein an sich bekanntes elektronisches Messverfahren angewendet, mit dem bisher die Bewegung des Unterkiefers in Bezug auf den Oberkiefer in drei Dimensionen bestimmt wird. Mit anderen Worten mit diesem Verfahren werden elektronische Signaldaten erzeugt und zur Darstellung der Bewegungen des Unterkiefers ausgewertet. Neben der Anwendung des bekannten elektronischen Messverfahrens wird eine statistische Auswertung einer Mehrzahl von Bewegungen des Unterkiefers in Bezug auf den Oberkiefer vorgenommen.

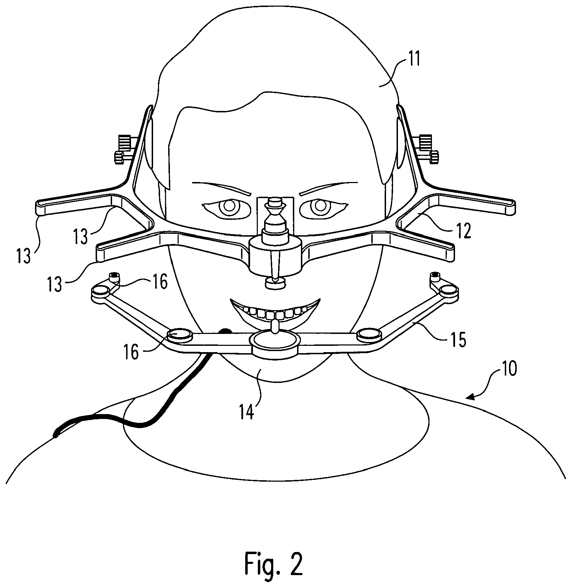

In Fig. 2 ist die neue Messanordnung zum Ermitteln der Zentrikposition gezeigt. Am oberen Teil des Kopfes 11 eines Patienten 10 ist ein Trägergestell 12 befestigt, welches vier Ausleger hat. Jeder Ausleger trägt zwei nach unten gerichtete Ultraschall-Mikrofone als Empfängerelemente. Mit dem Unterkiefer 14 des Patienten 10 ist ein weiteres Trägergestell 15 verbunden. Dieses ist mittels einer Bissgabel auf den Zähnen des Unterkiefers unter Verwendung einer Plastikmasse aufgeklebt. Das Gestell 15 trägt vier nach oben gerichtete Ultraschallsender als Geberelemente.

Bei dem bekannten Digma-Gerät wird mit drei Geberelementen und vier Empfängerelementen gearbeitet. Das ergibt zwölf Messstrecken, deren Längenveränderung bei der Bewegung des Unterkiefers ausgewertet wird. Es hat sich jedoch herausgestellt, dass die damit erzielbare Messgenauigkeit nicht ausreichend ist, um die Zentrikposition mit der nunmehr gewünschten Präzision zu ermitteln. Die Präzision wird jedoch durch die bei dem neuen Gerät vorgesehenen acht Geberelemente und vier Empfängerelemente erreicht, mit denen 32 Messstrecken ausgewertet werden können. Es sei jedoch an dieser Stelle betont, dass die Zahl der Geberelemente und der Empfängerelemente nur hinsichtlich der Präzision Auswirkungen haben, nicht jedoch entscheidend für die grundsätzliche Funktion der erfindungsgemäßen Verfahren sind.

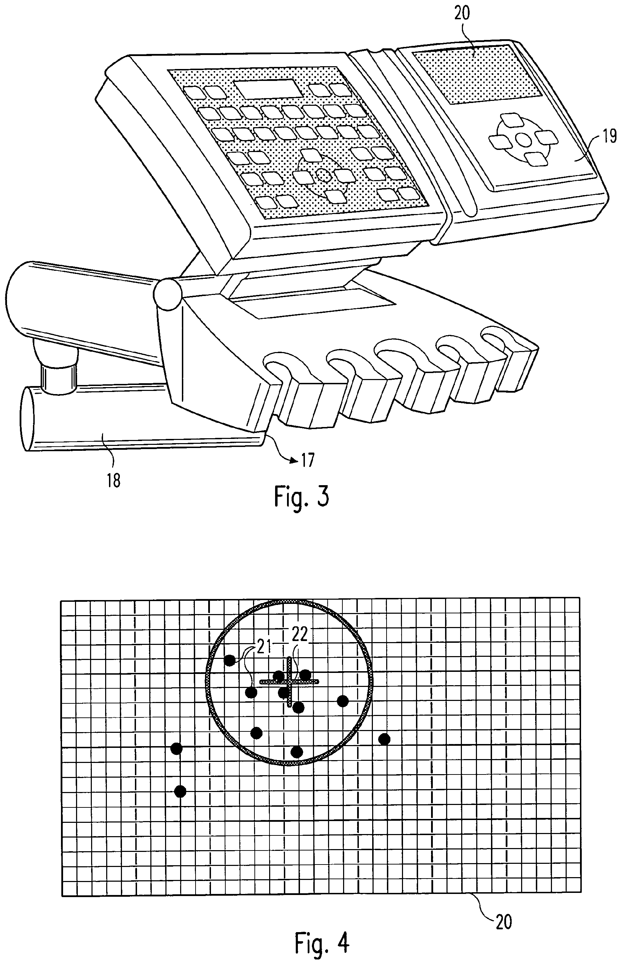

In Fig. 3 ist das neue Gerät 19 zur Ausführung der erfindungsgemäßen Verfahren gezeigt. Es ist an dem Ausleger 18 einer zahnärztlichen Behandlungseinheit 17 angedockt und weist ein Display 20 auf. Das Gerät 19 ist über Verbindungskabel oder kabellos mit den Geberelementen 16 und den Empfängerelementen 13 an den Trägergestellen 12 und 15 in Fig. 2 verbunden. Es misst die Bissendstellungen des Unterkiefers 14 in Form von Signaldaten.

Die Bissendstellungen mehrere aufeinanderfolgender Beißbewegungen des Patienten sind auf dem in Fig. 4 vergrößert dargestellten Display 20 in Form von Punkten 21 dargestellt. Das Display ist mit einem Gitternetz kartesischer Koordinaten überzogen. Man erkennt, dass die Bissendstellungen mehr oder weniger voneinander abweichen. Die innerhalb eines durch einen Kreis angedeuteten Trefferfeldes liegenden Punkte 21 werden zur Bestimmung der Zentrikposition ausgewertet. Eine Möglichkeit zur Auswertung ist eine Mittelwertbildung aus den Koordinaten aller Punkte 21, der im Trefferfeld liegt. Der Mittelwert wird durch das Kreuz 22 angezeigt und stellt die Zentrikposition dar. Aufgrund der Kenntnis der Zentrikposition 22 kann der Zahnarzt nunmehr den Patienten durch Führung von dessen Unterkiefer veranlassen, bei einer erneuten einmaligen Beißbewegung den Unterkiefer in die durch die Zentrikposition gekennzeichnete Bissendstellung zu bewegen und ein zwischen die Zähne gelegtes Registrat zu programmieren. Das so programmierte Registrat 9 wird dann zwischen die Zähen der beiden Gipsmodelle im Artikulator 4 (Fig. 1) eingelegt. Danach werden die Gelenke 4, 5 des Artikulators 4 in bekannter Weise so verstellt, dass die Zähne der Gipsmodelle 7, 8 formschlüssig in die entsprechenden Abdrücke in dem Registrat 9 eingreifen.

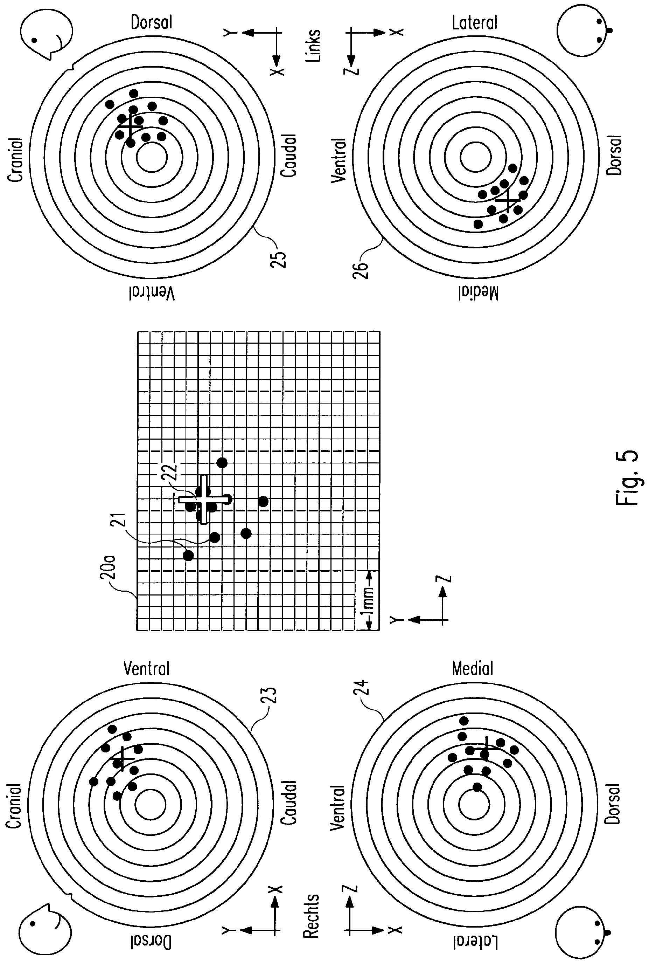

Neben oder anstellte der Signaldaten der ermittelten Zentrikposition können auch die Kieferngelenk-Positionen des Patienten ermittelt werden. Dies ist in Fig. 5 gezeigt. Der Bildschirm 20 zeigt hier ein Koordinatensystem 20a in verkleinerter Form, ebenfalls mit mehreren verschiedenen Bissendstellungen entsprechenden Punkten und der daraus ermittelten Zentrikposition 22. Neben der Darstellung 20a sind auf dem Bildschirm 20 jeweils links und rechts zwei Polarkoordinatenebenen 23, 24; 25, 26 dargestellt, die Schnitte durch den Kopf des Patienten repräsentieren. Die Schnitte sind durch die neben den Polarkoordinatenebenen befindlichen medizinischen Fachbezeichnungen (dorsal, kaudal, ventral, cranial, lateral und medial) sowie durch die stilisierten Kopfsymbole gekennzeichnet. Die Polarkoordinatenebenen 23-26 haben die Form von konzentrischen Ringen. Die der Zentrikposition 22 in der kartesischen Koordinatenebene 20a gezeigte Zentrikposition 22 entspricht den Polarkoordinaten-Punkten in den vier Polarkoordinatenebenen 23-26. Die mehreren Bissendstellungen ergeben auch in den Polarkoordinatenebenen eine entsprechende Anzahl von Punkten und durch die Auswertung von deren Position eine Zentrikposition in Form eines Kreuzes.

Statt nun - wie in Verbindung mit Fig. 4 erläutert wurde - die ermittelte Zentrikposition zum Programmieren eines Registrats zu verwenden, ist es möglich, die in Fig. 5 in den Polarkoordinatenebenen niedergelegten Zentrikpositionen der Kieferngelenke direkt, d.h. ohne Zwischenschaltung eines Registrats, zur Justage der Gelenke des Artikulators 1 zu verwenden. Dazu sollte an dem Artikulator ein Geber-Sensor-System angebracht sein, das demjenigen in Fig. 2 entspricht. In diesem Fall kann die die Justage der Kieferngelenke vornehmende Person sich am Display orientieren. Alternativ dazu ist es aber auch möglich, dass die Gelenk des Artikulators in drei Dimensionen motorisch verstellbar sind, so dass die Justage der Gelenkpositionen des Artikulators nicht von Hand erfolgen muss, sondern automatisch vorgenommen wird.

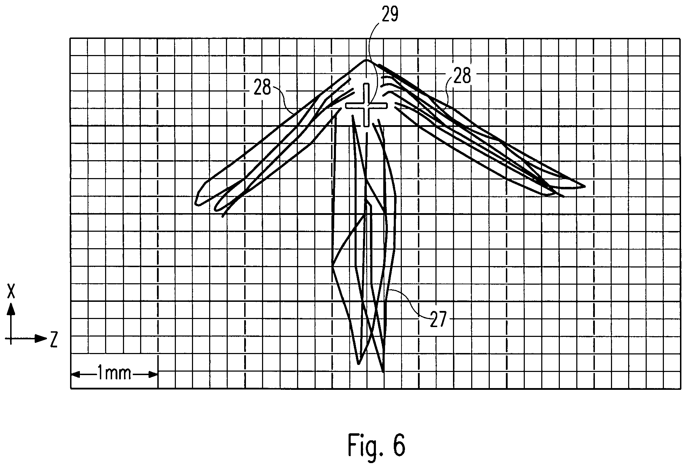

In Fig. 6 ist eine dritte Methode zur Ermittlung der Zentrikposition gezeigt. Hier ist wieder das Display 20 mit einem kartesischen Koordinatensystem gezeigt. Der Zahnarzt veranlasst den Patienten, den Unterkiefer bei vollständig oder weitgehend aufeinander liegenden Zähnen nach vorn und hinten sowie zu beiden Seiten zu bewegen. Auf diese Weise erscheint auf dem Bildschirm ein T-artiges Bewegungsmuster von Linien, wobei die Linien 27 die Vor- und Zurückbewegung des Unterkiefers und die Linien 28 die Seitenbewegungen des Unterkiefers repräsentieren. Der obere Schenkel des T ist durchhängend, d.h. spitz oder bogenförmig. Die Bestimmung der Zentrikposition erfolgt durch die Auswertung durch aller Bewegungslinien, bspw. durch Mittelwertbildung, wobei der Schnittpunkt zwischen den beiden T-Schenkeln als Zentrikposition 29 festgelegt wird, die hier wieder als Kreuz dargestellt ist. Diese Zentrikposition kann wiederum zur Programmierung eines Registrats verwendet werden oder aber in Verbindung mit den in Fig. 5 gezeigten Polarkoordinatenebenen zur direkten Justage des Artikulators.

## Patentansprüche

1. Verfahren zum Ermitteln der Zentrikposition eines menschlichen Gebisses, bei dem der Unterkiefer zur Ausführung einer Beißbewegung veranlasst wird, und bei der die Endstellung der Beißbewegung zur Ermittlung der Zentrikposition ausgewertet wird, wobei mehrere Beißbewegungen durchgeführt werden, dass die Beißbewegungen messtechnisch erfasst und in elektrische Signaldaten umgewandelt werden, und dass aus den Signaldaten mehrerer Beißendstellungen die Zentrikposition ermittelt wird, **dadurch gekennzeichnet, dass** je ein Trägergestell (12, 15) in einer reproduzierbaren Position mit dem Unterkiefer und dem Oberkiefer des betreffenden Patienten verbindbar ist und, dass die Entfernungen zwischen den Sender- und Empfängerelementen gemessen und zur Gewinnung der Signaldaten ausgewertet werden.

2. Verfahren nach Anspruch 1,
**dadurch gekennzeichnet,**
**dass** zur Ermittlung der Zentrikposition nur die in einer Koordinatenebene in einem begrenzten Trefferfeld liegenden Signaldaten ausgewertet werden.

3. Verfahren zum Ermitteln der Zentrikposition eines menschlichen Gebisses, bei dem der Unterkiefer mindestens eine Bewegung in Bezug auf den Oberkiefer ausführt, und bei dem mindestens zwei Bewegungen zur Ermittlung der Zentrikposition ausgewertet werden, wobei der Unterkiefer in der Beißendstellung zu mehreren zwischen einer vorderen und einer hinteren Endposition verlaufenden ersten Verschiebebewegungen sowie zu mehreren zwischen seitlichen Endpositionen verlaufenden zweiten Verschiebebewegungen veranlasst wird, wobei die ersten und zweiten Verschiebebewegungen messtechnisch erfasst und in elektrische Signaldaten umgewandelt werden, und wobei die Signaldaten dahingehend ausgewertet werden, wobei der Schnittpunkt der ersten und der zweiten Verschiebebewegungen die Zentrikposition ergibt, **dadurch gekennzeichnet, dass** je ein Trägergestell (12, 15) in einer reproduzierbaren Position mit dem Unterkiefer und dem Oberkiefer des betreffenden Patienten verbindbar ist und, dass die Entfernungen zwischen den Sender- und Empfängerelementen gemessen und zur Gewinnung der Signaldaten ausgewertet werden.

4. Verfahren nach einem der vorherigen Ansprüche,
**dadurch gekennzeichnet,**
**dass** anstelle oder neben den Signaldaten für die der Zentrikposition die Signaldaten für die Kieferngelenkpositionen ermittelt werden, und dass aus den Signaldaten für die Kiefergelenkpositionen die Zentrikposition für jedes der beiden Kiefergelenke ermittelt wird.

5. Verfahren nach Anspruch 3,
**dadurch gekennzeichnet,**
**dass** die Signaldaten der ersten und zweiten Verschiebebewegung sowie die Signaldaten der ermittelten Zentrikposition auf einem Display als Symbole, wie z.B. Punkte, dargestellt werden.

6. Verfahren nach Anspruch 5,
**dadurch gekennzeichnet,**
**dass** die Symbole auf dem Display in einer Koordinatenebene dargestellt sind.

7. Verfahren nach Anspruch 6,
**dadurch gekennzeichnet,**
**dass** die Koordinaten kathesische Koordinaten sind.

8. Verfahren nach einem der vorherigen Ansprüche,
**dadurch gekennzeichnet,**
**dass** der Unterkiefer nach Feststellung der Zentrikposition anhand der ermittelten Signaldaten gesteuert in die Zentrikposition bewegt wird, so dass die Zähne von Ober- und Unterkiefer in einem zwischen diese eingelegtes plastisches Registrat Abdrücke produzieren, die die Informationen über die Zentrikposition und die entsprechenden Kiefergelenkpositionen enthält.

9. Verfahren nach Anspruch 1,
**dadurch gekennzeichnet,**
**dass** die Signaldaten der Beißendstellungen aller Beißbewegungen und die Signaldaten der ermittelten Zentrikposition auf einem Display als Symbole, wie z.B. Punkte, dargestellt werden.

10. Verfahren nach Anspruch 9,
**dadurch gekennzeichnet,**
**dass** die Symbole auf dem Display in einer Koordinatenebene dargestellt sind, die die tatsächlichen Abstände der Beißendstellungen von der Zentrikposition in entsprechenden Maßeinheiten erkennen lässt.

11. Verfahren nach Anspruch 10,
**dadurch gekennzeichnet,**
**dass** die Koordinaten karthesische Koordinaten sind.

12. Verfahren nach einem der vorherigen Ansprüche 4 bis 11,
**dadurch gekennzeichnet,**
**dass** die Signaldaten der Zentrikposition der Kiefergelenke auf einem Display als Symbole, wie z.B. Punkte, in je zwei Koordinatenebenen dargestellt werden, und die Koordinatenebenen einen Winkel, vorzugsweise einen solchen von 90°, einschließen.

13. Verfahren nach Anspruch 12,
**dadurch gekennzeichnet,**
**dass** die eine Koordinatenebene diejenige Schnittebene durch den Patientenkopf repräsentiert, die einerseits zwischen oben (cranial) und unten (cardial) und andererseits vorn (ventral) und hinten (dorsal) verläuft, und die andere Koordinatenebene diejenige Schnittebene durch den Patientenkopf repräsentiert, die einerseits zwischen vorn (ventral) und hinten (dorsal) und andererseits zwischen der Körperachse (medial) und der entsprechenden Seite (lateral) verläuft.

14. Verfahren nach Anspruch 12 oder 13,
**dadurch gekennzeichnet,**
**dass** die Koordinaten nach Art von Polarkoordinaten in Form von konzentrischen Ringen dargestellt werden.

15. Verfahren nach Anspruch 8,
**dadurch gekennzeichnet,**
**dass** das Registrat mit den Abdrücken in einen Artikulator eingesetzt wird, um zwecks Präparation eines Zahnersatzes darin befindliche Modelle des Oberkiefers und des Unterkiefers de Patienten zueinander auszurichten.

16. Verfahren nach einem der vorherigen Ansprüche 4 bis 15,
**dadurch gekennzeichnet,**
**dass** die Signaldaten der Zentrikposition der Kieferngelenke zur direkten Justage der Genlenke des Artikulators oder zur Steuerung von mit dem Artikulator zusammenwirkenden motorischen Antrieben verwendet werden.

## Claims

1. A method for the determination of the centric position of a human set of teeth, in which the lower jaw is made to carry out a biting movement, and in which the end position of the biting movement is evaluated for the determination of the centric position,
wherein
a plurality of biting movements are performed, the biting movements are detected by means of measuring techniques and converted into electrical signal data, and the centric position is determined from the signal data of a plurality of bite end positions,
**characterised in that**
in each case one carrier frame (12, 15) is connectable in a reproducible position with the lower jaw and the upper jaw of the patient concerned, and the distances between the transmitter and receiver elements are measured and evaluated for the extraction of the signal data.

2. A method according to claim 1,
**characterised in that**
only the signal data lying in a coordinate plane in a restricted hit field are evaluated for the determination of the centric position.

3. A method for the determination of the centric position of a human set of teeth, in which the lower jaw carries out at least one movement in relation to the upper jaw and in which at least two movements are evaluated for the determination of the centric position,
wherein
the lower jaw in the bite end position is made to carry out a plurality of first displacement movements running between a forward and a rearward end position as well as a plurality of second displacement movements running between lateral end positions, wherein the first and second displacement movements are detected by means of measuring techniques and converted into electrical signal data, and wherein the signal data are evaluated to the effect that the point of intersection between the first and the second displacement movements yields the centric position,
**characterised in that**
in each case one carrier frame (12, 15) is connectable in a reproducible position with the lower jaw and the upper jaw of the patient concerned, and the distances between the transmitter and receiver elements are measured and evaluated for the extraction of the signal data.

4. A method according to one of the preceding claims,
**characterised in that**
instead of or besides the signal data for the centric position the signal data for the jaw-joint positions are determined, and from the signal data for the jaw-joint positions the centric position is determined for each of the two jaw joints.

5. A method according to claim 3,
**characterised in that**
the signal data of the first and second displacement movement as well as the signal data of the centric position determined are represented on a display as symbols, such as e.g. points.

6. A method according to claim 5,
**characterised in that**
the symbols are represented on the display in a coordinate plane.

7. A method according to claim 6,
**characterised in that**
the coordinates are Cartesian coordinates.

8. A method according to one of the preceding claims,
**characterised in that**
after ascertainment of the centric position on the basis of the signal data determined, the lower jaw is moved in a controlled manner into the centric position so that the teeth of the upper and lower jaw produce impressions in a plastic register placed between them containing the information about the centric position and the corresponding jaw-joint positions.

9. A method according to claim 1,
**characterised in that**
the signal data of the bite end positions of all the biting movements and the signal data of the centric position determined are represented on a display as symbols, such as e.g. points.

10. A method according to claim 9,
**characterised in that**
the symbols are represented on the display in a coordinate plane which indicates the actual distances of the bite end positions from the centric position in corresponding measurement units.

11. A method according to claim 10,
**characterised in that**
the coordinates are Cartesian coordinates.

12. A method according to one of the preceding claims 4 to 11,
**characterised in that**
the signal data of the centric position of the jaw joints are represented on a display as symbols, such as e.g. points, in, in each case, two coordinate planes, and the coordinate planes enclose an angle, preferably an angle of 90°.

13. A method according to claim 12,
**characterised in that**
the one coordinate plane represents that sectional plane through the patient's head which, on the one hand, runs between the top (cranial) and the bottom (cardial) and, on the other hand, between the front (ventral) and the rear (dorsal), and the other coordinate plane represents that sectional plane through the patient's head which, on the one hand, runs between the front (ventral) and the rear (dorsal) and, on the other hand, between the body axis (medial) and the corresponding side (lateral).

14. A method according to claim 12 or 13,
**characterised in that**
the coordinates are represented in the manner of polar coordinates in the form of concentric rings.

15. A method according to claim 8,
**characterised in that**
the register with the impressions is inserted into an articulator in order, for the purpose of preparation of a denture, to align models of the upper jaw and the lower jaw of the patient located therein to one another.

16. A method according to one of the preceding claims 4 to 15,
**characterised in that**
the signal data of the centric position of the jaw joints are used for the direct adjustment of the joints of the articulator or for the control of motor drives cooperating with the articulator.

## Revendications

1. Procédé servant à déterminer la position centrale d'une dentition humaine, dans lequel la mâchoire inférieure est amenée à exécuter un mouvement de serrage des dents, et dans lequel la position finale lors du mouvement de serrage de dents est évaluée afin de déterminer la position centrale,
dans lequel
plusieurs mouvements de serrage des dents sont effectués, les mouvements de serrage des dents sont détectés par une technique de mesure et sont convertis en des données de signaux électriques, et la position centrale est déterminée à partir des données de signaux de plusieurs positions finales lors du serrage des dents,
**caractérisé en ce que**
respectivement un châssis de support (12, 15) peut être relié, dans une position reproductible, à la mâchoire inférieure et la mâchoire supérieure du patient concerné, et que
les éloignements entre les éléments d'émission et de réception sont mesurés et sont évalués afin d'obtenir les données de signaux.

2. Procédé selon la revendication 1,
**caractérisé en ce**
**que** seules les données de signaux se trouvant dans un plan de coordonnées dans un champ d'impact délimité sont évaluées afin de déterminer la position centrale.

3. Procédé servant à déterminer la position centrale d'une dentition humaine, dans lequel la mâchoire inférieure exécute au moins un mouvement par rapport à la mâchoire supérieure, et dans lequel au moins deux mouvements sont évalués afin de déterminer la position centrale,
dans lequel
la mâchoire inférieure dans la position finale de serrage des dents est amenée à réaliser plusieurs premiers mouvements de coulissement s'étendant entre une position finale avant et une position finale arrière ainsi que plusieurs deuxièmes mouvements de coulissement s'étendant entre des positions finales latérales, dans lequel les premiers et les deuxièmes mouvements de coulissement sont détectés par une technique de mesure et sont convertis en des données de signaux électriques, et dans lequel
les données de signaux sont évaluées à ce sujet, dans lequel
le point de coupe des premiers et des deuxièmes mouvements de coulissement donne la position centrale,
**caractérisé en ce que**
respectivement un châssis de support (12, 15) peut être relié, dans une position reproductible, à la mâchoire inférieure et la mâchoire supérieure du patient concerné, et que
les éloignements entre les éléments d'émission et les éléments de réception sont mesurés et sont évalués afin d'obtenir les données de signaux.

4. Procédé selon l'une quelconque des revendications précédentes,
**caractérisé en ce**
**qu'**en lieu et place ou outre les données de signaux de la position centrale, les données de signaux sont déterminées pour les positions d'articulations des mâchoires, et que la position centrale pour chacune des deux articulations de mâchoire est déterminée à partir des données de signaux pour les positions d'articulations de mâchoires.

5. Procédé selon la revendication 3,
**caractérisé en ce**
**que** les données de signaux des premiers et deuxièmes mouvements de coulissement ainsi que les données de signaux de la position centrale déterminée sont affichées sur un écran sous la forme de symboles, par exemple de points.

6. Procédé selon la revendication 5,
**caractérisé en ce**
**que** les symboles sont affichés sur l'écran dans un plan de coordonnées.

7. Procédé selon la revendication 6,
**caractérisé en ce**
**que** les coordonnées sont des coordonnées cartésiennes.

8. Procédé selon l'une quelconque des revendications précédentes,
**caractérisé en ce**
**que** la mâchoire inférieure est déplacée de manière commandée dans la position centrale une fois la position centrale relevée à l'aide des données de signaux déterminées, de sorte que les dents de la mâchoire supérieure et inférieure produisent, dans un registre plastique intercalé entre ces dernières, des empreintes, qui contiennent les informations sur la position centrale et les positions d'articulations de mâchoires correspondantes.

9. Procédé selon la revendication 1,
**caractérisé en ce**
**que** les données de signaux des positions finales de serrage des dents de tous les mouvements de serrage des dents et les données de signaux de la position centrale déterminée sont affichées sur un écran sous la forme de symboles, par exemple de points.

10. Procédé selon la revendication 9,
**caractérisé en ce**
**que** les symboles sont affichés sur l'écran dans un plan de coordonnées, qui laisse apparaître les écarts réels entre les positions finales de serrage des dents et la position centrale dans des unités de mesure correspondantes.

11. Procédé selon la revendication 10,
**caractérisé en ce**
**que** les coordonnées sont des coordonnées cartésiennes.

12. Procédé selon l'une quelconque des revendications précédentes 4 à 11,
**caractérisé en ce**
**que** les données de signaux de la position centrale des articulations de mâchoires sont affichées sur un écran sous la forme de symboles, par exemple de points, dans respectivement deux plans de coordonnées, et les plans de coordonnées forment un angle, de préférence un angle de 90°.

13. Procédé selon la revendication 12,
**caractérisé en ce**
**que** l'un plan de coordonnées représente le plan de coupe à travers la tête du patient, qui s'étend d'une part entre le haut (crânial) et le bas (cardial) et d'autre part entre l'avant (ventral) et l'arrière (dorsal), et l'autre plan de coordonnées représente le plan de coupe à travers la tête du patient, qui s'étend d'une part entre l'avant (ventral) et l'arrière (dorsal) et d'autre part entre l'axe du corps (médial) et le côté correspondant (latéral).

14. Procédé selon la revendication 12 ou 13,
**caractérisé en ce**
**que** les coordonnées sont affichées à la manière de coordonnées polaires sous la forme d'anneaux concentriques.

15. Procédé selon la revendication 8,
**caractérisé en ce**
**que** le registre comprenant les empreintes est inséré dans un articulateur pour, aux fins de la préparation d'une prothèse dentaire, orienter les unes par rapport aux autres des modélisations se trouvant dans cette dernière de la mâchoire supérieure et de la mâchoire inférieure du patient.

16. Procédé selon l'une quelconque des revendications précédentes 4 à 15,
**caractérisé en ce**
**que** les données de signaux de la position centrale des articulations des mâchoires sont utilisées aux fins de l'ajustement direct des articulations de l'articulateur ou aux fins de la commande d'entraînements à moteur coopérant avec l'articulateur.
